(19) Europäisches Patentamt
European Patent Office
Office européen des brevets

(11) Numéro de publication: **0 007 839**
**B1**

(12) **FASCICULE DE BREVET EUROPEEN**

(45) Date de publication du fascicule de brevet: **02.09.81**

(21) Numéro de dépôt: **79400468.9**

(22) Date de dépôt: **06.07.79**

(51) Int. Cl.³: **C 07 D 333/32,**
C 07 D 307/58,
C 07 D 331/04,
C 07 D 305/12,
C 07 D 335/02,
C 07 D 309/32
//C07D409/04

(54) **Nouveau procédé de préparation d'acides cyclopropane-1-carboxyliques 3-substitués et composés nouveaux obtenus.**

(30) Priorité: **24.07.78 FR 7821813**

(43) Date de publication de la demande:
**06.02.80 Bulletin 80/3**

(45) Mention de la délivrance du brevet:
**02.09.81 Bulletin 81/35**

(84) Etats Contractants Désignés:
**AT CH DE FR GB IT NL SE**

(56) Documents cités:
**FR - A - 2 232 273**
**FR - A - 2 268 806**
**FR - A - 2 396 006**

(73) Titulaire: **ROUSSEL-UCLAF**
**102, route de Noisy Boîte postale no.9**
**F-93230 Romainville (FR)**

(72) Inventeur: **Martel, Jacques**
**15, rue Douvillez**
**F-93140-Bondy (FR)**
Inventeur: **Tessier, Jean**
**30, rue Jean Moulin**
**F-94300-Vincennes (FR)**
Inventeur: **Demoute, Jean-Pierre**
**249bis, rue de Rosny**
**F-93100 Montreuil sous Bois (FR)**

(74) Mandataire: **Douetteau, Pierre et al,**
**Boîte postale no 9 102, route de Noisy**
**F-93230-Romainville (FR)**

**EP 0 007 839 B1**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

Courier Press, Leamington Spa, England.

Nouveau procédé de préparation d'acides cyclopropane-1-carboxyliques 3-substitués et composés nouveaux obtenus.

La présente invention concerne un nouveau procédé de préparation d'acides cyclopropane-1-carboxyliques 3-substitués et des composés nouveaux obtenus.

L'invention a pour objet un procédé de préparation des acides de formule générale (I) de configuration (1R,3S):

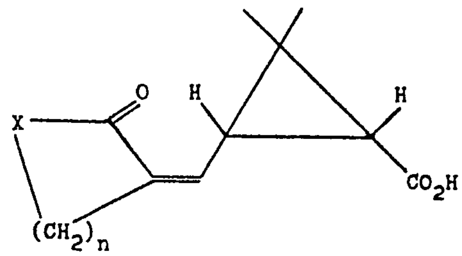

(I)

dans laquelle X représente un atome de soufre ou d'oxygène et n représente un nombre entier égal à 1, 2 ou 3, caractérisé en ce que l'on fait réagir, au sein d'un solvant organique, en présence d'un agent basique fort, la lactone de l'acide cis 2,2-diméthyl 3S-(dihydroxyméthyl)cyclopropane-1R-carboxylique(II):

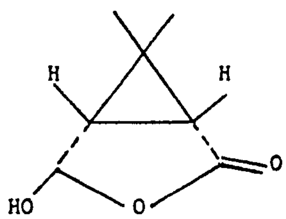

(II)

avec un composé de formule générale (III):

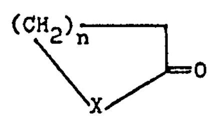

(III)

dans laquelle X et n sont définis comme précédemment, soumet le composé résultant de formule générale (IV):

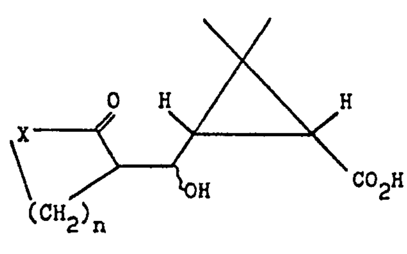

(IV)

à l'action d'un agent acide, au sein d'un solvant organique apolaire, puis soumet le composé résultant de formule générale (V):

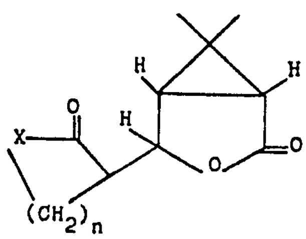

(V)

à l'action d'un agent basique au sein d'un solvant organique apolaire pour obtenir le composé (I) correspondant désiré.

L'invention a notamment pour objet un procédé tel que défini précédemment, caractérisé en ce que X représente un atome de soufre.

L'agent basique fort est choisi avantageusement dans le groupe constitué par les alcoolates alcalins, les amidures alcalins, les hydrures alcalins et les métaux alcalins.

Comme solvant organique au sein duquel on fait réagir la lactone (II) et le composé (III), on utilise, notamment, le tétrahydrofuranne.

L'agent acide est choisi avantageusement dans le groupe constitué par l'acide paratoluène sulfonique, l'acide benzène sulfonique, l'acide chlorhydrique et l'acide sulfurique.

Le solvant organique apolaire au sein duquel on fait réagir l'agent acide est, notamment, un hydrocarbure aromatique, tel que le benzène ou le toluène.

Comme agent basique on peut choisir avantageusement une base tertiaire telle que la triéthylamine ou la pyridine.

Le solvant organique apolaire au sein duquel on fait réagir l'agent basique est, notamment, un hydrocarbure aromatique tel que ceux cités plus haut.

L'invention a, en particulier, pour objet, un procédé de préparation selon la méthode précédemment indiquée, concernant les composés pour lesquels n=2 et X est un atome de soufre, caractérisé en ce que l'on fait réagir au sein de tétrahydrofuranne, en présence de terbutylate de potassium, la lactone de l'acide cis 2,2-diméthyl 3S-(dihydroxyméthyl) cyclopropane-1R-carboxylique avec la dihydro 3-(2H)-thiophénone soumet l'acide (1R,3S) 3-/(dihydro 2-oxo-3 (2H) thiényl) hydroxyméthyl/ 2,2-diméthyl cyclopropane-1-carboxylique résultant, au sein du benzène, à l'action de l'acide paratoluène sulfonique, puis fait réagir la triéthylamine au sein du benzène sur l'acide (1R,4R,5S) 4-(dihydro 2-oxo-3 (2H) thiényl) 6,6-diméthyl 3-oxabicyclo/3.1.0/ hexan-2-one résultant, pour obtenir l'acide (1R,3S) 3-/(dihydro 2-oxo-3 (2H) thiénylidène) méthyl/ 2,2-diméthyl cyclopropane-1-carboxylique désiré.

D'une manière analogue, en utilisant la 2-thiétanone ou la tétrahydro (2H)-thiopyran-2-one à la place de la dihydro-2-(3H)-thiophénone, on obtient respectivement l'acide (1R,3S) 3-/(2-oxo 3-thiétannylidène)méthyl/ 2,2-diméthyl cyclopropane-1-carboxylique ou l'acide (1R,3S) 3-/(2-oxo tétrahydro 3-(2H)thiopyrannylidène) méthyl/ 2,2-diméthyl cyclopropane-1-carboxylique.

D'un manière analogue, en utilisant la 2-oxétanone, la dihydro-2-(3H) furanone ou la tétrahydro (2H) pyran-2-one à la place de la dihydro-2-(3H)-thiophénone, on obtient respectivement l'acide (1R,3S) 3-/(2-oxo 3-oxétannylidène)-méthyl/2,2-diméthyl cyclopropane-1-carboxylique, l'acide(1R,3S) 3-/(dihydro-2-oxo-3-(2H)-furannylidène)méthyl/ 2,2-diméthyl cyclopropane-1-caboxylique ou l'acide (1R,3S) 3-/(2-oxo-tétrahydro-3-(2H)-pyrannylidène)méthyl/ 2,2-diméthyl cyclopropane-1-carboxylique.

—l'acide (1R,3S) 3-/(2-oxo 3-thiétannylidène)méthyl/ 2,2-diméthyl cyclopropane-1-carboxylique, l'acide (1R,3S) 3-/(2-oxo tétrahydro 3-(2H) thiopyrannylidène méthyl/ 2,2-diméthyl cyclopropane-1-carboxylique, l'acide (1R,3S) 3-/(2-oxo 3-oxétannylidène)méthyl/ 2,2-diméthyl cyclopropane-1-carboxylique et l'acide (1R,3S) 3-/(2-oxo tétrahydro-3-(2H) pyrannylidène)méthyl/ 2,2-diméthyl cyclopropane-1-carboxylique sont des produits nouveaux et à ce titre, font partie de l'invention.

Les composés de formule I peuvent être utilisés pour la préparation d'esters insecticides.

On connaissait déjà un procédé de préparation de l'acide (1R,3S) 3/(dihydro 2-oxo-3 (2H) furannylidène) méthyl/ 2,2-diméthyl cyclopropane-1-carboxylique consistant à effectuer une réaction de Wittig en faisant réagir l'acide 2,2-diméthyl 3S-formyl cycylopropane-1R-carboxylique avec le 2-oxo 3-oxa cyclopentylidène triphényl phosphorane (BF: 2.045.177).

On connaissait de même par le brevet français 2.097.244, un procédé de préparation de l'acide (1R,3S) 3/(dihydro 2-oxo 3-(2H) thiénylidène)méthyl/ 2,2-diméthyl cyclopropane-1-carboxylique, consistant à effectuer une réaction de Wittig en faisant réagir l'acide 2,2-diméthyl 3S-formyl cyclopropane-1R-carboxylique avec le 2-oxo 3-thia cyclopentylidène triphényl phosphorane.

La présente invention fournit un procédé plus avantageux que le procédé cité précédemment.

En effet, dans le procédé de l'invention, les composés intermédiaires peuvent ne pas être isolés, le mode opératoire correspondant est alors particulièrement simple et facile à industrialiser.

Les réactifs utilisés dans le procédé de l'invention sont facilement accessibles ce qui n'est pas le cas du phosphorane utilisé dans les brevets cités ci-dessus.

D'autre part le rendement global du procédé de l'invention est nettement plus élevé que celui du procédé décrit dans les brevets français précites. Il est à noter, en particulier, que la condensation du composé (III) et de la lactone (II) s'effectue avec un rendement très élevé et dans des conditions extrément douces que n'étaient pas, à priori, prévisibles. En général, en effet, les lactones ou thiolactones du type (III) ne réagissent pas facilement avec un groupement aldéhydique tel que celui qui existe (sous forme lactonisée) dans la lactone (II). Il est connu que, pour que ce type de réaction entre un composé du type (III) et un composé porteur d'une fonction aldéhyde s'effectue facilement et fournisse un rendement élevé en composé du type IV, il est nécessaire que la fonction aldéhyde soit fixée sur un noyau aromatique.

La première étape du procédé de l'invention possède donc, sur le plan chimique, un caractère inattendu qui mérite d'être signalé.

L'exemple suivent illustre l'invention sans toutefois la limiter.

Exemple

*Acide (1R,3S)-3/(dihydro 2-oxo 3(2H) thiénylidène) méthyl/-2,2-diméthyl cyclopropane-1-carboxylique.*

STADE A: Acide (1R,3S)-3/(dihydro 2-oxo 3(2H)thienyl) hydroxyméthyl/-2,2-diméthyl cyclopropane-1-carboxylique.

Dans 50 cm3 de tétrahydrofuranne, on introduit 2,84 g de lactone de l'acide cis 2,2-dimethyl 3S (dihydroxyméthyl) cyclopropane-1R-carboxylique, 2,2 g de dihydro 3-(2H)-thiophénone, refroidit à —60°C., sous atmosphère inerte, introduit, en 30 minutes environ, une solution de 4,5 g de terbutylate de potassium dans 35cm3 de tétrahydrofuranne anhydre, agite pendant 1 heure à —60°C., ajoute lentement sans que la température n s'élève au dessus de —20°C, 50 cm3 de solution aqueuse normale d'acide chlorhydrique, dilue ensuite abondamment à l'eau, extrait au chlorure de méthylène, lave à l'eau les extraits réunis, sèche sur sulfate de sodium, filtre, concentre à sec par distillation et obtient 6,2 g d'acide (1R,3S)-3/(dihydro 2-oxo 3(2H) thienyl) hydroxyméthyl/-2,2-diméthyl cyclopropane-1-carboxylique brut, utilisé tel que pour le stade suivant.

STADE B: (1R,4R,5S) 4-(dihydro 2-oxo-3(2H) thienyl)-6,6-diméthyl 3-oxabicyclo (3.1.0) hexan-2-one.

On dissout les 6,2 g de composé hydroxylé obtenus au stade A précédent dans 70 cm3 de benzène, ajoute 50 mg d'acide paratoluène sulfonique, porte au reflux pendant 1h30 en éliminant l'eau formée par passage du solvant condensé sur gel de silice. On obtient une solution contenant la (1R,4R,5S) 4-(dihydro 2-oxo-3(2H) thiènyl)-6,6-diméthyl 3-oxabicyclo (3.1.0) hexan-2-one, utilisée, telle quelle pour le stade suivant.

STADE C: Acide (1R,3S)-3/(dihydro-2-oxo-3(2H) thiénylidène) méthyl/-2,2-diméthyl cyclopropane-1-carboxylique.

A la solution obtenue au stade B précédent, on ajoute 6 cm3 de triéthylamine, porte le mélange au reflux, l'y maintient pendant 3 heures, refroidit, verse le mélange réactionnel sur une solution aqueuse 2N d'acide chlorhydrique, sépare par décantation le phase organique, la lave à l'eau, la séche et la concentre par distillation. Le produit brut est repris à chaud par 20 cm3 de chlorure de méthylène, on ajoute 30 cm3 d'heptane normal puis refroidit à +5°C, on essore le précipité formé, le lave à l'heptane, le sèche et obtient 3,025 g d'acide (1R,3S)-3/(dihydro 2-oxo-3(2H) thiénylidène) méthyl/-2,2-diméthyl cyclopropane-1-carboxylique. F=162°C.

*Spectre de R.M.N. (deutérochloroforme)*

Pics à 1,29—1,38 p.p.m. attribués aux hydrogènes de méthyles en position 2 du cyclopropyle;
Pics à 1,83—2,08 p.p.m. attribués aux hydrogènes en positions 1 et 3 du cyclopropyle;
Pics à 2,83—3,5 p.p.m. attribués aux hydrogènes du cyclopentyle;
Pics à 6,77—6,92 p.p.m. attribués à l'hydrogène en position 1' de la chaîne latérale éthylique.

**Revendications**

1. Procédé de préparation des acides de formule générale (I) de configuration (1R,3S):

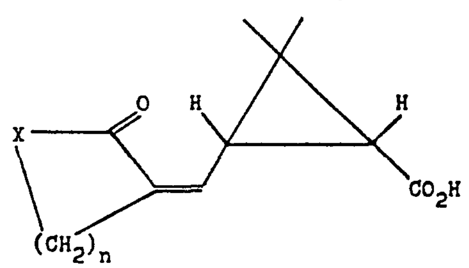

(I)

dans laquelle X représente un atome de soufre ou d'oxygène et n représente un nombre entier égal à 1, 2 ou 3, caractérisé en ce que l'on fait réagir, au sein d'un solvant organique, en présence d'un agent basique fort, la lactone de l'acide cis 2,2-diméthyl 3S-(dihydroxy méthyl) cyclopropane-1R-carboxylique (II):

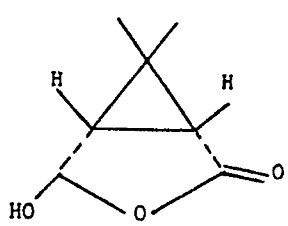

(II)

avec un composé de formule générale (III):

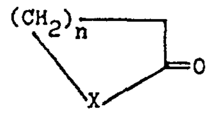

(III)

dans laquelle X et n sont définis comme ci-dessus, soumet le composé résultant de formule générale (IV):

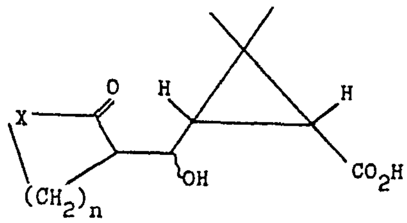

(IV)

à l'action d'un agent acide, au sein d'un solvant organique apolaire, puis soumet le composé résultant de formule générale (V):

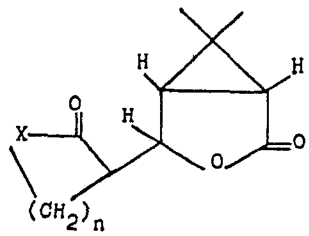

(V)

à l'action d'un agent basique au sein d'un solvant organique apolaire pour obtenir le composé (I) correspondant désiré.

2. Procédé selon la revendication 1, caractérisé en ce que X représente un atome de soufre.

3. Procédé de préparation selon la revendication 1 ou 2, caractérisé en ce que l'agent basique fort est choisi dans le groupe constitué par les alcoolates alcalins, les amidures alcalins, les hydrures alcalins et les métaux alcalins.

4. Procédé de préparation selon l'une quelconque des revendications 1 à 3, caractérisé en ce que le solvant organique au sein duquel on fait réagir la lactone (II) et le composé (III) est le tétrahydrofuranne.

5. Procédé de préparation selon l'une quelconque des revendications 1 à 4, caractérisé en ce que l'agent acide est choisi dans le groupe constitué par l'acide paratoluène sulfonique, l'acide benzène sulfonique, l'acide chlorhydrique et l'acide sulfurique et le solvant organique apolaire est un hydrocarbure aromatique.

6. Procédé de préparation selon l'une quelconque des revendications 1 à 5, caractérisé en ce que l'agent basique est une base tertiaire telle que la triethylamine ou la pyridine et le solvant organique apolaire est un hydrocarbure aromatique.

7. Procédé de préparation selon l'une quelconque des revendications 1 à 6, caractérisé en ce que n=2.

8. Procédé selon l'une quelconque des revendications 3 à 7, caractérisé en ce que X représente un atome de soufre.

9. Composés de formule (I) telle que définie à la revendication 1, caractérisés en ce que, dans ladite formule I, n=1 ou n=3.

**Patentansprüche**

1. Verfahren zur Herstellung von Säuren der allgemeinen Formel (I) mit (1R,3S)-Konfiguration:

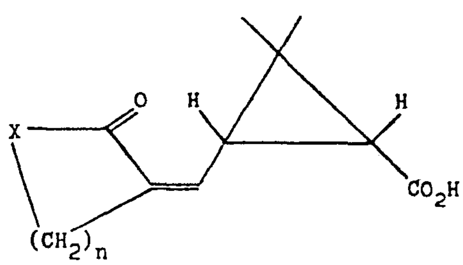

(I)

worin X ein Schwefel- oder Sauerstoffatom bedeutet und n eine ganze Zahl, nämlich 1, 2 oder 3 ist, dadurch gekennzeichnet, daß man in dem Medium eines organischen Lösungsmittels in Gegenwart eines starken basischen Mittels das Lacton der cis-2,2-Dimethyl-3S-(dihydroxymethyl)-cyclopropan-1R-carbonsäure (II):

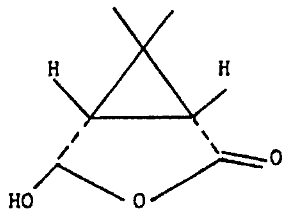

(II)

mit einer Verbindung der allgemeinen Formel (III):

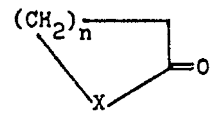

(III)

worin X und n die vorstehend angegebenen Bedeutungen besitzen, umsetzt, die erhaltene Verbindung der allgemeinen Formel (IV):

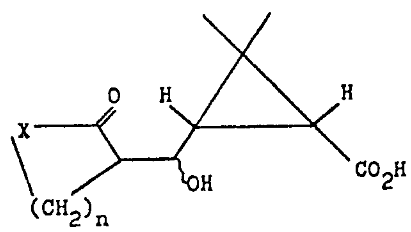

(IV)

in dem Medium eines apolaren organischen Lösungsmittels der Einwirkung eines sauren Mittels unterzieht und anschließend die erhaltene Verbindung der allgemeinen Formel (V):

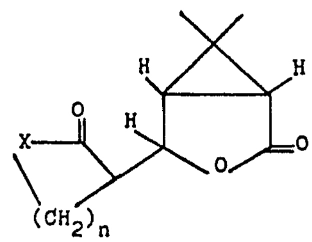

(V)

der Einwirkung eines basischen Mittels in dem Medium eines apolaren organischen Lösungsmittels unterzieht, um die entsprechende gewünschte Verbindung (I) zu erhalten.

2. Verfahren gemäß Anspruch 1, dadurch gekennzeichnet, daß X ein Schwefelatom bedeutet.

3. Verfahren gemäß Anspruch 1 oder 2, dadurch gekennzeichnet, daß das starke basische Mittel ausgewählt wird unter den Alkalialkoholaten, den Alkaliamiden, den Alkalihydriden und den Alkalimetallen.

4. Verfahren gemäß einem der Ansprüche 1 bis 3, dadurch gekennzeichnet, daß das organische Lösungsmittel, in dessen Medium man das Lacton (II) und die Verbindung (III) umsetzt, Tetrahydrofuran ist.

5. Verfahren gemäß einem der Asprüche 1 bis 4, dadurch gekennzeichnet, daß die Säure ausgewählt wird unter p-Toluolsulfonsäure, Benzosulfonsäure, Chlorwasserstoffsäure und Schwefelsäure und daß das apolare organische Lösungsmittel ein aromatischer Kohlenwasserstoff ist.

6. Verfahren gemäß einem der Ansprüche 1 bis 5, dadurch gekennzeichnet, daß das basische Mittel eine tertiäre Base, wie Triäthylamin oder Pyridin, ist und daß das apolare organische Lösungsmittel ein aromatischer Kohlenwasserstoff ist.

7. Verfahren gemäß einem der Ansprüche 1 bis 6, dadurch gekennzeichnet, daß n=2.

8. Verfahren gemäß einem der Ansprüche 3 bis 7, dadurch gekennzeichnet, daß X ein Schwefelatom bedeutet.

9. Verbindungen der Formel (I) gemäß Anspruch 1, dadurch gekennzeichnet, daß in der Formel I n=1 oder n=3.

**Claims**

1. Process for preparing the acids of general formula (I) of configuration (1R,3S):

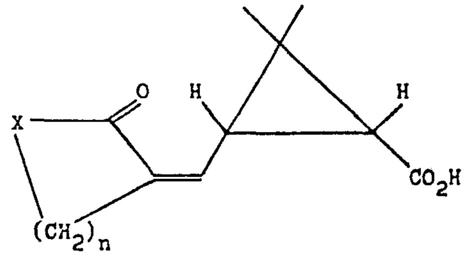

(I)

in which X represents a sulphur or oxygen atom and *n* represents an integer equal to 1, 2 or 3, characterised in that in an organic solvent, in the presence of a strong basic agent, the lactone of *cis* 2,2-dimethyl 3S-(dihydroxy methyl) cyclopropane-1R-carboxylic acid (II):

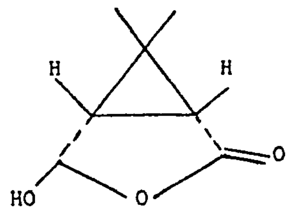

(II)

is reacted with a compound of general formula (III):

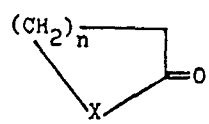

(III)

in which X and *n* are defined as above, the resultant compound of general formula (IV):

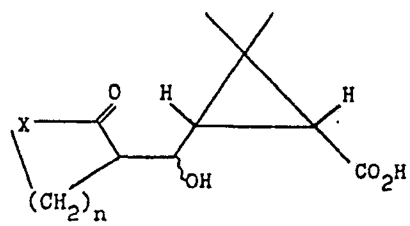

(IV)

is subjected to the action of an acid agent, in an apolar organic solvent, then the resultant compound of general formula (V):

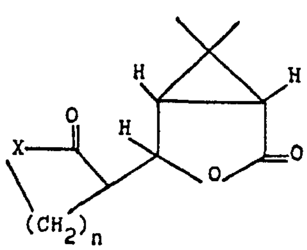

(V)

is subjected to the action of a basic agent in an apolar organic solvent to obtain the desired corresponding compound (I).

2. Process according to Claim 1, characterised in that X represents a sulphur atom.

3. Preparation process according to Claim 1 or 2, characterised in that the strong basic agent is selected from the group constituted by the alkali alcoholates, the alkali amides, the alkali hydrides and the alkali metals.

4. Preparation process according to any one of Claims 1 to 3, characterised in that the organic solvent in which the lactone (II) and the compound (III) are reacted is tetrahydrofuran.

5. Preparation process according to any one of Claims 1 to 4, characterised in that the acid agent is selected from the group constituted by paratoluene sulphonic acid, benzene sulphonic acid, hydrochloric acid and sulphuric acid and the apolar organic solvent is an aromatic hydrocarbon.

6. Preparation process according to any one of Claims 1 to 5, characterised in that the basic agent is a tertiary base such as triethylamine or pyridine and the apolar organic solvent is an aromatic hydrocarbon.

7. Preparation process according to any one of Claim 1 to 6, characterised in that $n=2$.

8. Process according to any one of Claims 3 to 7, characterised in that X represents a sulphur atom.

9. Compounds of formula (I) as defined in Claim 1, characterised in that, in the said formula I. $n=1$, or $n=3$.